# EUROPÄISCHE PATENTSCHRIFT

(11) **EP 0 722 924 B1**
(45) Veröffentlichungstag und Bekanntmachung des Hinweises auf die Patenterteilung: **01.07.1998**
(21) Anmeldenummer: 96100157.5
(22) Anmeldetag: 08.01.1996
(51) Int. Cl.: C07C 51/567, C07C 55/10

(54) **Verfahren zur Herstellung von Bernsteinsäureanhydrid**
Process for the preparation of succinic anhydride
Procédé pour la préparation de l'anhydride succinique

(30) Priorität: 20.01.1995 DE 19501676
(43) Veröffentlichungstag der Anmeldung: 24.07.1996
(73) Patentinhaber: BAYER AG, 51368 Leverkusen (DE)
(72) Erfinder: Darsow, Gerhard, Dr., D-47804 Krefeld (DE)

(56) Entgegenhaltungen:
- DE-B- 1 226 556
- US-A- 2 198 153

## Beschreibung

Die vorliegende Erfindung betrifft ein kostengünstiges, kontinuierlich arbeitendes Verfahren zur Herstellung von Bernsteinsäureanhydrid, bei welchem nur sehr geringe Mengen des üblicherweise bei der Hydrierung von Maleinsäureanhydrid als Neenprodukt entstehenden γ-Butyrolactons sowie keine Mono- und keine Hydroxycarbonsäuren der C-Zahlen <4 gebildet werden.

Bernsteinsäureanhydrid ist ein wichtiges Ausgangsprodukt für die Herstellung von thermoplastischen Polyestern, die besondere mechanische und chemische Eigenschaften sowie eine gute biologische Abbaubarkeit aufweisen.

Es ist bekannt, Bernsteinsäureanhydrid aus Bernsteinsäure diskontinuierlich durch eine Dehydratisierungsreaktion durch Einleiten von Essigsäureanhydrid-Dämpfen in geschmolzene Bernsteinsäure herzustellen (GB 507 592, zitiert nach Ullmann, Enzyklopädie der technischen Chemie, 3. Aufl. (1953), Bd. 4, S. 318).

Es ist ferner bekannt, Maleinsäureanhydrid im Batch-Prozeß über Ni (US 2 198 153, zitiert nach Ullmann, Enzyklopädie der technischen Chemie, 3. Aufl. (1953), Bd. 4, S. 318) oder Pd, Rh, Pt/Al₂O₃ (JP 48/7609 (1973)) zu Bernsteinsäureanhydrid zu hydrieren.

Es ist weiterhin bekannt, Maleinsäureanhydrid über Pd/A-Kohle kontinuierlich zu Bernsteinsäureanhydrid zu hydrieren (SU 721 406).

Der Reaktionsverlauf läßt sich durch das folgende Reaktionsschema veranschaulichen:

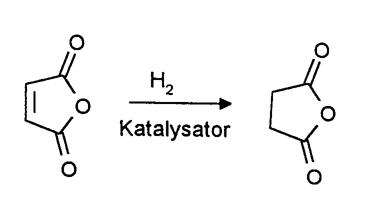

Bei den bekannten Verfahren zur Herstellung von Bernsteinsäureanhydrid werden überwiegend diskontinuierliche Suspensionsverfahren (Batch-Prozesse) angewendet, bei denen das Maleinsäureanhydrid mit und ohne Lösungsmittel über pulverförmigen Katalysatoren mit Wasserstoff hydriert wird.

Diskontinuierliche Verfahren haben den Nachteil, daß ihre Kapazität relativ zum Reaktionsvolumen sehr klein ist und somit ein Bedarf nach großen Reaktionsapparaturen und Lagertanks besteht. Energieverbrauch und Personalbedarf sind verhältnismäßig hoch.

Kontinuierliche Pulverkatalysatorverfahren, die mit mehreren in Kaskade geschalteten Hydrierreaktoren arbeiten, vermeiden einen Teil dieser Nachteile. Es bleibt jedoch das Erfordernis, die pulverförmigen Katalysatoren mehrfach gezielt zu dosieren, umzupumpen und quantitativ vom Reaktionsprodukt abzufiltrieren. Die Katalysatorschlammpumpen unterliegen einem hohen mechanischen Verschleiß. Die quantitative Entfernung der pulverförmigen Katalysatoren aus dem Reaktionsprodukt ist aufwendig. Ferner ist die Gefahr groß, die Katalysatoraktivität durch die zusätzlichen Operationen verhältnismäßig schnell zu verringern. Es ist daher vorteilhaft, die Reaktion über fest angeordneten Katalysatoren ablaufen zu lassen. Solche Katalysatoren müssen eine hohe Aktivität besitzen, die über einen längeren Zeitraum nicht nachlassen darf, weil häufige Katalysatorwechsel bei Festbettreaktionen ebenfalls aufwendig sind.

Als kontinuierlich arbeitendes Verfahren wurde bisher die Hydrierung von Maleinsäureanhydrid zu Bernsteinsäureanhydrid über Pd/A-Kohle beschrieben. Dieser Katalysator hat nur eine begrenzte Lebensdauer. Außerdem kann die Reaktion nur mit Hilfe eines Lösungsmittels durchgeführt werden.

Überraschenderweise wurde nun gefunden, daß man Maleinsäureanhydrid sehr gut kontinuierlich über im Festbett angeordneten trägerfreien Formkörpern aus sauerstofffreien Metallpulvern von einem oder mehreren Elementen der Eisenuntergruppe der VIII. Nebengruppe des Periodensystems (Mendelejew) zu Bernsteinsäureanhydrid hydrieren kann. Dazu kann es nützlich sein, die Metalle der Eisenuntergruppe mit aktivierend wirkenden Elementen der VI. Nebengruppe des Periodensystems zu legieren. Dabei können die zum Einsatz kommenden Pulver zusätzlich kleine Anteile nicht katalytisch wirkender Elemente (z.B. Silizium, Aluminium, Titan, Kohlenstoff) enthalten, ohne daß die hohe Aktivität gemindert wird. Die Festkörper müssen eine Druckfestigkeit von 20-250 N und eine innere Oberfläche von 10-90 m²/g aufweisen.

Gegenstand der Erfindung ist damit ein Verfahren zur kontinuierlichen Herstellung von Bernsteinsäureanhydrid durch katalytische Hydrierung von Maleinsäureanhydrid, das dadurch gekennzeichnet ist, daß die Hydrierung in der flüssigen Phase bei einem H₂-Druck von 50-400 bar, einer 10-80-fachen molaren H₂-Menge, bezogen auf die stöchiometrische Menge, und bei einer Temperatur von 60-180°C an im Festbett angeordneten sauerstofffreien und trägerfreien Katalysatoren durchgeführt wird, die als verpreßte, aus Metallpulvern hergestellte Formkörper vorliegen, die eine Druckfestigkeit von 20-250 N und eine innere Oberfläche von 10-90 m²/g aufweisen und bei denen die Metallpulver 60-100 Gew.-% eines oder mehrerer Eisenmetalle, 0 - 15 Gew.-% eines oder mehrerer Metalle der VI. Nebengruppe und 0-25 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

Die Druckfestigkeit der trägerfreien Formkörper kann nach DIN 50 106 bestimmt werden.

Die Überprüfung von trägerfreien Formkörpern auf die anspruchsgemäßen inneren Oberflächen und damit auf Brauchbarkeit für das erfindungsgemäße Verfahren können nach Methoden durchgeführt werden, die von F.M. Nelsen und F.T. Eggertsen, Analyt. Chem. 30 (1958), S. 1387-1390 bzw. S.J. Gregg und K.S.W. Sing, Adsorption, Surface Area and Porosity, London 1982, Kap. 2 und 6, beschrieben worden sind.

Die Eisenuntergruppe der VIII. Nebengruppe des Periodensystems (Mendelejew) enthält die Elemente Eisen, Kobalt und Nickel. Die erfindungsgemäß zu verwendenen trägerfreien Formkörper enthalten eines oder mehrere dieser Metalle in Mengen von mindestens 60, vorzugsweise mindestens 70, insbesondere mindestens 80 Gew.-%, bezogen auf das Gesamtgewicht der trägerfreien Formkörper.

Die VI. Nebengruppe des Periodensytems enthält die Elemente Chrom, Molybdän und Wolfram. Die erfindungsgemäß zu verwendenden trägerfreien Formkörper enthalten eines oder mehrerer dieser Metalle in Mengen von 0 - 15 Gew.-%. Für den Fall ihres Vorliegens enthalten die Metallpulver mindestens 0,1, vorzugsweise mindestens 0,3, insbesondere mindestens 0,5 Gew.-%, bezogen auf trägerfreie Formkörper; sie enthalten eines oder mehrere dieser Metalle in Mengen von höchstens 15, vorzugsweise höchstens 10 und insbesondere höchstens 5 Gew.-%, bezogen auf trägerfreie Formkörper.

Die erfindungsgemäß zu verwendenen trägerfreien Formkörper können darüber hinaus - jeweils bezogen auf trägerfreie Formkörper - bis zu 25, vorzugsweise bis zu 15 Gew.-% andere Elemente enthalten; Beispiele solcher Elemente, die nicht katalytisch wirken, umfassen Aluminium, Silicium, Titan und Kohlenstoff. Nach einer bevorzugten Ausführungsform enthalten die trägerfreien Formkörper außer den Metallen der VIII. und VI. Nebengruppe nicht mehr als 10 Gew.-% Aluminium und nicht mehr als 5 Gew.-% anderer Elemente.

Für den Hydrierprozeß wird auf einen Druck von 50 - 400 bar, bevorzugt 100 bis 400 bar, besonders bevorzugt 150-300 bar, vorkomprimierter reiner Wasserstoff eingesetzt, wobei man mit einer 20- bis 60-fachen, bevorzugt 20- bis 40-fachen molaren Wasserstoffmenge, bezogen auf die stöchiometrische Menge, arbeitet.

Die Hydrierung erfolgt kontinuierlich im Festbettverfahren an den als Hydrierungskatalysatoren dienenden trägerfreien Formkörpern der beschriebenen Art, indem man das zu hydrierende flüssige Maleinsäureanhydrid entweder im Gleichstrom mit dem zuvor zugemischten Wasserstoff von unten nach oben aufsteigend über die in den Hydrierreaktor gefüllten Formkörper strömen läßt oder auch dem von oben einströmenden Wasserstoff von unten kommend entgegenführt (Gegenstromverfahren). Das erfindungsgemäße Verfahren kann selbstverständlich auch in Lösungsmitteln durchgeführt werden. Geeignete unter den Reaktionsbedingungen inerte Lösungsmittel sind beispielsweise Di-n-propyl-ether, Di-iso-propyl-ether, Di-n-butyl-ether, Di-iso-butyl-ether, Tetrahydrofuran, Dioxan, Pyran, γ-Butyrolacton In bevorzugter Weise wird das erfindungsgemäße Verfahren ohne Lösungsmittel oder in γ-Butyrolacton als einem systemeigenen Lösungsmittel durchgeführt.

Der Hydrierprozeß wird bei Temperaturen von 60 bis 180°C, bevorzugt 80-160°C, besonders bevorzugt 100-140°C, durchgeführt. Niedrigere Temperaturen bedingen höhere Verweilzeiten oder den Verzicht auf einen quantitativen Umsatz. Höhere Temperaturen führen zur vermehrten Bildung von γ-Butyrolacton als Nebenprodukt.

Die stündliche Katalysatorbelastung kann 200 bis 500 g Maleinsäureanhydrid/l Katalysator betragen.

Zum Einsatz kommt Maleinsäureanhydrid mit einer Reinheit >99%. Es läßt sich aber auch Destillationsrückläufe (γ-Butyrolacton) enthaltendes Maleinsäureanhydrid einsetzen.

Der Hydrierreaktor kann entweder ein einzelnes Hochdruckrohr aus Stahl oder einer Stahllegierung sein, das mit den trägerfreien Formkörpern ganz oder teilweise gefüllt wird, wobei auch die Anwendung auf Horden (Drahtkörbe o.ä.) nützlich sein kann, oder aber ein ummanteltes Hochdruckrohrbündel, dessen Einzelrohre mit Formkörpern ganz oder teilweise gefüllt werden.

Die Herstellung der trägerfreien Formkörper kann nach gebräuchlichen Methoden durch Verpressen der Metallpulver auf Tablettier- und Pelletiermaschinen unter hohem Druck erfolgen, wobei zur Verbesserung des Haftvermögens der Metallpartikel auch Graphit in Mengen von 0,5 - 1,5 Gew.-%, bezogen auf das Gesamtgewicht der den Katalysator bildenden Bestandteile, oder Klebestoffe in kleinen Mengen zum Einsatz kommen können. Die Herstellung der trägerfreien Formkörper erfolgt vorzugsweise in einer sauerstofffreien Atmosphäre, um Oberflächenoxidationen zu vermeiden. Am wirksamsten und für die Reaktionsführung am günstigsten sind tablettierte und pelletierte Formkörper mit Durchmessern von 3 bis 7 mm. Von erheblicher Bedeutung ist die Druckfesfigkeit dem Formkörper, die erfindungsgemäß bei Werten von 20 bis 250 N, bevorzugt 100 bis 220 N, liegt. Niedrigere Druckfestigkeiten führen zu Formkörperzerfall bzw. erosivem Abrieb, was eine metallische Kontaminierung des Reaktionsproduktes bewirken würde. Höhere Werte bedingen einen unangemessenen Aufwand beim Verpressen, ohne daß weitere Vorteile erzielt werden. Von erheblicher Bedeutung ist weiterhin die innere Oberfläche der Formkörper, die erfindungsgemäß bei Werten von 10 bis 90 m²/g liegt und ausschlaggebend für einen möglichst quantitativen Umsatz der Einsatzstoffe ist.

Unter den geschilderten Reaktionsbedingungen sind auf diese Weise ganz unerwartet hohe Katalysatorstandzeiten von 15 000 Stunden und mehr zu erzielen, was zu Katalysatorverbräuchen < 0,1 Gew.-%, bezogen auf hergestelltes Reaktionsprodukt, führt.

Das den Hydrierreaktor verlassende Reaktionsgemisch wird entspannt, wobei man den überschüssigen Wasserstoff abfangen und nach erfolgtem Komprimieren und Ergänzung von verbrauchtem Wasserstoff erneut zum Einsatz bringen kann. Bei einer kompletten Hydrierung (99.9-100%iger Umsatz des Maleinsäureanhydrids) besteht das Reaktionsgemisch zu mindestens 97 Gew.-% aus Bernsteinsäureanhydrid. Es kann an organischen Leichtsiedern bis zu 3 Gew.-% γ-Butyrolacton enthalten.

Die erfindungsgemäß einzusetzenden sauerstofffreien und trägerfreien Festbettkatalysatoren neigen im Gegensatz zu trägerhaltigen Katalysatoren nicht zum "Ausbluten", d.h. nicht zum Übergang von Katalysatorbestandteilen in ionischer oder kolloidaler Form in die Lösungsphase des Substrats, so daß das Substrat nicht durch Schwermetalle kontaminiert wird, die normalerweise ebenfalls nur mühsam, beispielsweise mit Hilfe von Ionenaustauschern, aus dem Substrat erntfernt werden können. Die einzusetzenden Katalysatormetalle können, etwa nach längerem Gebrauch des Katalysators leicht aufgearbeitet und wiederverwendet werden, da die Schwermetalle nicht umständlich von einem Trägermaterial getrennt werden müssen.

Das erzeugte Bernsteinsäureanhydrid weist einen Gehalt an Katalysatorbestandteilen von <1 ppm auf, wird nach der destillativen Entfernung des Leichtsieders in einer Reinheit von ≥ 99,9 Gew.-% erhalten und ist daher ohne jede weitere Reinigung zur weiteren Verwendung, beispielsweise auch zur Herstellung von Polymeren einsetzbar.

Die nach der Destillation erhaltene farblose und glasklare Schmelze von Bernsteinsäureanhydrid kann entweder in Kristallisationsgeräten üblicher Bauart zur Kristallisation gebracht oder über Abschuppwalzen zu rieselfähigen Schuppen verarbeitet werden.

### Beispiele

### Beispiel 1

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Nickelpulver hergestellten Hydrierungskatalysators gefüllt, der bei einer Zylinderhöhe von 5 mm und einem Durchmesser von 5 mm eine Druckfestigkeit von 147 N auf die Zylindermantelfläche und eine innere Oberfläche von 33 m²/g aufwies. Durch dieses Rohr wurden gemeinsam mit der 20fachen molaren Menge von unter einem Druck von 300 bar stehendem, hochreinem Wasserstoff stündlich 420 g einer in einem Schmelzkessel hergestellten glasklaren Schmelze von Maleinsäureanhydrid kontinuierlich gepumpt und zwar von unten nach oben aufsteigend.

Maleinsäureanhydridschmelze und Wasserstoff wurden vorab gemeinsam durch einen Wärmeaustauscher geführt und so hoch erhitzt, daß sie in das Hochdruckrohr mit einer Temperatur von 130°C eintraten. Das das Hochdruckrohr verlassende Gemisch aus flüssigem Reaktionsprodukt und überschüssigem Wasserstoff wurde in einen Abscheider geführt, von wo der Wasserstoff nach Ersatz der verbrauchten Menge wieder zusammen mit neuer Maleinsäureanhydridschmelze in den Vorwärmer und von da erneut in das Hochdruckrohr gepumpt wurde.

Die farblose und klare Schmelze des Reaktionsproduktes wurde nach Entspannung auf Normaldruck und Abkühlung gaschromatographisch untersucht.

Sie enthielt an organischen Leichtsiedern 1,5 Gew.-% γ-Butyrolacton, so daß der Bernsteinsäureanhydridgehalt des organischen Reaktionsproduktes bei 98,5 Gew.-% lag.

Das erzeugte Bernsteinsäureanhydrid wurde nach der destillativen Entfernung des Leichtsieders in einer Reinheit von 99,9 Gew.-% erhalten.

Der Katalysator war nach einer Laufzeit von 4.100 Stunden unverändert wirksam, so daß sich die Zusammensetzung des Reaktionsproduktes über diesen Zeitraum nicht veränderte.

### Beispiel 2

In einem Hochdruckrohr wie in Beispiel 1 wurde bei einer Temperatur von 125°C und einem Wasserstoffdruck von 200 bar der Wasserstoff im umgekehrten Reaktionsfluß wie in Beispiel 1 der aufsteigenden Maleinsäureanhydridschmelze entgegengeführt, wobei stündlich eine gleichgroße Menge wie in Beispiel 1 hydriert wurde. Der Katalysator war durch Tablettierung einer pulverisierten Nickel-Eisenlegierung gewonnen worden. Die Legierung enthielt einen Eisenanteil in Nickel von 15 Gew.-%. Die Tabletten hatten bei einer Zylinderhöhe von 5 mm und einem Durchmesser von 5 mm eine Druckfestigkeit von 137 N auf die Zylindermantelfläche und eine innere Oberfläche von 74 m²/g.

Nach einer Laufzeit von 3.200 Stunden lag der Umsatz des eingesetzten Maleinsäureanhydrids bei 99,95 Gew.-%. Der Gehalt an γ-Butyrolacton im Reaktionsprodukt lag bei 1,85 Gew.-%, so daß der Bernsteinsäureanhydridgehalt des Reaktionsproduktes bei 98,10 Gew.-% lag (Rest = 0,05 Gew.-% nicht umgesetzes Maleinsäureanhydrid).

Nach der destillativen Entfernung der Verunreinigungen wurde das erzeugte Bernsteinanhydrid in einer Reinheit von 99,9 Gew.-% erhalten.

### Beispiel 3

Ein senkrechtes stehendes, wärmeisoliertes Hochdruckrohr aus nichtrostendem Stahl von 45 mm Innendurchmesser und 1 m Länge wurde mit 1,4 l eines durch Tablettierung von Pulver einer Ni/Mo-Legierung mit einem Mo-Gehalt von 1,75% hergestellten Hydrierungskatalysator gefüllt, der bei einer Zylinderhöhe von 5 mm und einem Durchmesser von 5 mm eine Druckfestigkeit von 191 N und eine innere Oberfläche von 58 m²/g aufwies. Durch dieses Rohr wurden gemeinsam mit der dreißigfachen molaren Menge von unter einem Druck von 300 bar stehendem hochreinem Wasserstoff stündlich 560 g Maleinsäureanhydridschmelze gepumpt und zwar von unten nach oben aufsteigend.

Maleinsäureanhydridschmelze und Wasserstoff wurden vor Eintritt in das Hochdruckrohr auf eine Temperatur von 120°C gebracht.

Nach einer Laufzeit von 1.980 Stunden lag der Umsatz des eingesetzten Maleinsäureanhydrids bei 100 Gew.-%. Der Gehalt an γ-Butyrolacton im Reaktionsprodukt lag bei 1,07 Gew.-%, so daß der Bernsteinsäureanhydridgehalt bei 98,93 Gew.-% lag.

Nach der destillativen Entfernung der Verunreinigung wurde das gewonnene Bernsteinsäureanhydrid in einer Reinheit von 99,9 Gew.-% erhalten.

### Beispiel 4

In einem Hochdruckrohr wie in Beispiel 1, aber aus Hochdruckstahl N 9, wurde bei einer Temperatur von 120°C und einem Wasserstoffdruck von 300 bar stündlich eine gleichgroße Menge Maleinsäureanhydrid hydriert. Der Katalysator wurde durch Tablettierung von Pulver einer Ni/Mo/Al-Legierung mit einem Mo-Gehalt von 1,02 Gew.-% und einem Al-Gehalt von 6,1 Gew.-% hergestellt. Die Tabletten hatten bei einer Zylinderhöhe von 5 mm und einem Durchmesser von 5 mm eine Druckfestigkeit von 210 N und eine innere Oberfläche von 71 m²/g.

Nach einer Laufzeit von 2.800 Stunden lag der Gehalt an Bernsteinsäureanhydrid im Reaktionsprodukt bei 98,9 Gew.-% und der Gehalt an γ-Butyrolacton bei 1,1 Gew.-%.

### Beispiel 5

In einem Hochdruckrohr wie in Beispiel 1 wurde bei einer Temperatur von 125°C und einem Wasserstoffdruck von 300 bar stündlich eine gleichgroße Menge Maleinsäureanhydrid hydriert. Der Katalysator wurde durch Tablettierung einer pulverisierten Ni/Fe/Mo-Legierung gewonnen. Die Legierung enthielt einen Fe-Anteil von 15% sowie einen Mo-Gehalt von 1,4%. Die Tabletten hatten bei einer Zylinderhöhe von 3 mm und einem Durchmesser von 3 mm eine Druckfestigkeit von 162 N und eine innere Oberfläche von 68 m²/g.

Nach einer Laufzeit von 1.700 Stunden lag der Umsatz des eingesetzten Maleinsäureanhydrids bei 99,95 Gew.-%. Der Gehalt an γ-Butyrolacton im Reaktionsprodukt betrug 1.27 Gew.-%, so daß der Bernsteinsäureanhydridgehalt des Reaktionsproduktes bei 98,68 Gew.-% lag (Rest = 0,05 Gew.-% nicht umgesetzes Maleinsäureanhydrid).

### Beispiel 6

In einem Hochdruckrohr wie in Beispiel 1 wurde bei einer Temperatur von 130°C und einem Wasserstoffdruck von 300 bar stündlich eine Menge von 420 g Maleinsäureanhydrid hydriert. Der Katalysator wurde durch Tablettierung einer pulverisierten Ni/Al/Si-Legierung mit einem Al-Gehalt von 5,4 Gew.-% und einem Si-Gehalt von 0,2 Gew.-% gewonnen.

Die Tabletten hatten bei einer Zylinderhöhe von 3 mm und einem Durchmesser von 3 mm eine Druckfestigkeit von 148 N und eine innere Oberfläche von 61 m²/g.

Nach einer Laufzeit von 1.400 Stunden lag der Umsatz des eingesetzten Maleinsäureanhydrids bei 99,9 Gew.-%. Der Gehalt an γ-Butyrolacton im Reaktionsprodukt betrug 1,0 Gew.-%, so daß der Bernsteinsäureanhydridgehalt bei 98,9 Gew.-% lag (Rest = 0,1 Gew.-% nicht umgesetztes Maleinsäureanhydrid).

### Beispiel 7

In einem Hochdruckrohr wie in Beispiel 1 wurde bei einer Temperatur von 120°C und einem Wasserstoffdruck von 300 bar stündlich eine Menge von 200 g Maleinsäureanhydrid, gelöst als 30 Gew.-%ige Lösung in γ-Butyrolacton, hydriert. Der Katalysator wurde durch Tablettierung einer pulverisierten Ni/Al-Legierung mit einem Al-Gehalt von 6,1 Gew.-% hergestellt.

Die Tabletten hatten bei einer Zylinderhöhe von 3 mm und einem Durchmesser von 3 mm eine Druckfestigkeit von 156 N auf die Zylindermantelfläche und eine innere Oberfläche von 69 m²/g.

Nach einer Laufzeit von 1.680 Stunden lag der Umsatz des eingesetzten Maleinsäureanhydrids bei 99,9 Gew.-%.

Nach der destillativen Entfernung des Lösungsmittels und einer danach durchgeführten Destillation des rohen Bernsteinsäureanhydrids hatte dieses eine Reinheit von 99,9 Gew.-%. Das abdestillierte γ-Butyrolacton wurde wieder in den Prozeß zurückgeführt.

## Patentansprüche

1. Verfahren zur kontinuierlichen Herstellung von Bernsteinsäureanhydrid durch katalytische Hydrierung von Maleinsäureanhydrid, dadurch gekennzeichnet, daß die Hydrierung in der flüssigen Phase bei einem H₂-Druck von 50 bis 400 bar, einer 10 bis 80-fachen molaren H₂-Menge, bezogen auf die stöchiometrische Menge, und bei einer Temperatur von 60 bis 180°C an im Festbett angeordneten sauerstofffreien und trägerfreien Katalysatoren durchgeführt wird, die als verpreßte aus Metallpulvern hergestellte Formkörper vorliegen, die eine Druckfestigkeit von 20 bis 250 N und eine innere Oberfläche von 10 bis 90 m²/g aufweisen und bei denen die Metallpulver 60 bis 100 Gew.-% eines oder mehrerer Eisenmetalle, 0 bis 15 Gew.-% eines oder mehrerer Metalle der VI. Nebengruppe und 0 bis 25 Gew.-% eines oder mehrerer hydrierinerter Elemente aus der Gruppe von Aluminium, Silicium, Titan und Kohlenstoff, alles bezogen auf das Gesamtgewicht des Metallpulvers, enthalten.

2. Verfahren nach Anspruch 1, dadurch gekennzeichnet, daß die Metallpulver 70 bis 100 Gew.-%, bevorzugt 80 bis 100 Gew.-%, eines oder mehrerer Eisenmetalle enthalten.

3. Verfahren nach Anspruch 1, dadurch gekennzeichnet, daß die Metallpulver beim Vorliegen von Metallen der VI. Nebengruppe einen Gehalt von 0,1 bis 15 Gew.-%, bevorzugt von 0,3 bis 10 Gew.-%, besonders bevorzugt von 0.5 bis 5 Gew.-%, daran enthalten.

4. Verfahren nach Anspruch 1, dadurch gekennzeichnet,, daß die Metallpulver beim Vorliegen von hydrierinerten Elementen einen Gehalt von 0 bis 10 Gew.-% an Aluminium und von 0 bis 5 Gew.-% je Element Si, Ti und C enthalten.

5. Verfahren nach Anspruch 4, dadurch gekennzeichnet, daß der Gesamtgehalt der hydrierinerten Elemente 0 bis 15 Gew.-%, bevorzugt 0 bis 10 Gew.-%, beträgt.

6. Verfahren nach Anspruch 1, dadurch gekennzeichnet, daß es sich bei den Formkörpern um solche mit einer Druckfestigkeit von 100 bis 220 N handelt.

7. Verfahren nach Anspruch 1, dadurch gekennzeichnet, daß die Formkörper zylinder- oder kugelförmig sind und Durchmesser von 3 bis 7 mm besitzen.

8. Verfahren nach Anspruch 1, dadurch gekennzeichnet, daß die Hydrierung bei einem H₂-Druck von 100 bis 400 bar, bevorzugt 150-300 bar, durchgeführt wird.

9. Verfahren nach Anspruch 1, dadurch gekennzeichnet, daß bei einer 20 bis 80-fachen molaren H₂-Menge gearbeitet wird.

10. Verfahren nach Anspruch 1, dadurch gekennzeichnet, daß das Maleinsäureanhydrid den Hydrierreaktor von unten nach oben aufsteigend passiert, während der für die Hydrierung benötigte Wasserstoff entweder gemeinsam mit dem ungesättigten Ester in den Reaktor gepumpt wird oder diesem, von oben nach unten strömend, entgegengeführt wird.

## Claims

1. Process for the continuous preparation of succinic anhydride by catalytic hydrogenation of maleic anhydride, characterized in that the hydrogenation is carried out in the liquid phase at an H₂ pressure of 50 to 400 bar, a 10 to 80 times molar amount of H₂, based on the stoichiometric amount, and at a temperature of 60 to 180°C and on oxygen-free support-free catalysts which are arranged in the fixed bed and are present as pressed shaped bodies which are produced from metal powders and have a compressive strength of 20 to 250 N and an internal surface area of 10 to 90 m²/g and in which the metal powders contain 60 to 100 % by weight of one or more ferrous metals, 0 to 15 % by weight of one or more metals of subgroup VI and 0 to 25 % by weight of one or more hydrogenation-inert elements from the group consisting of aluminium, silicon, titanium and carbon, all based on the total weight of metal powder.

2. Process according to Claim 1, characterized in that the metal powders contain 70 to 100 % by weight, preferably 80 to 100 % by weight, of one or more ferrous metals.

3. Process according to Claim 1, characterized in that the metal powders, when metals of subgroup VI are present, have a content of 0.1 to 15 % by weight, preferably of 0.3 to 10 % by weight, particularly preferably 0.5 to 5 % by weight, thereof.

4. Process according to Claim 1, characterized in that the metal powders, when hydrogenation-inert elements are present, have a content of 0 to 10 % by weight of aluminium and of 0 to 5 % by weight per element of Si, Ti and C.

5. Process according to Claim 4, characterized in that the total content of the hydrogenation-inert elements is 0 to 15 % by weight, preferably 0 to 10 % by weight.

6. Process according to Claim 1, characterized in that the shaped bodies are those having a compressive strength of 100 to 220 N.

7. Process according to Claim 1, characterized in that the shaped bodies are cylindrical or spherical and have diameters of 3 to 7 mm.

8. Process according to Claim 1, characterized in that the hydrogenation is carried out at an H₂ pressure of 100 to 400 bar, preferably 150-300 bar.

9. Process according to Claim 1, characterized in that a 20 to 80 times molar amount of H₂ is employed.

10. Process according to Claim 1, characterized in that the maleic anhydride passes through the hydrogenation reactor ascending from bottom to top, while the hydrogen required for the hydrogenation is either pumped into the reactor together with the unsaturated ester or is conducted in the opposite direction to this, flowing from top to bottom.

## Revendications

1. Procédé pour la préparation en continu d'anhydride succinique par hydrogénation catalytique d'anhydride maléique, caractérisé en ce qu'on effectue l'hydrogénation en phase liquide sous une pression de H₂ de 50 à 400 bar, avec une quantité de H₂ de 10 à 80 fois molaire rapportée à la quantité stoechiométrique et à une température de 60 à 180°C sur des catalyseurs en lit fixe, exempts d'oxygène et sans support, qui sont présents sous forme de corps moulés comprimés préparés à partir de poudres métalliques, qui présentent une résistance à la pression de 20 à 250 N et une surface interne de 10 à 90 m²/g, et dans lesquels les poudres métalliques contiennent, à concurrence de 60 à 100% en poids, un ou plusieurs métaux choisis parmi le fer et sa famille, à concurrence de 0 à 15% en poids, un ou plusieurs métaux du VI^{ème} sous-groupe et, à concurrence de 0 à 25% en poids, un ou plusieurs éléments inertes vis-à-vis de l'hydrogénation, choisis parmi le groupe comprenant l'aluminium, le silicium, le titane et le carbone, toutes ces teneurs se rapportant au poids total de la poudre métallique.

2. Procédé selon la revendication 1, caractérisé en ce que les poudres métalliques contiennent, à concurrence de 70 à 100% en poids, de préférence de 80 à 100% en poids, un ou plusieurs métaux choisis parmi le fer et sa famille.

3. Procédé selon la revendication 1, caractérisé en ce que la teneur des poudres métalliques en métaux du VI^{ème} sous-groupe, lorsque ces derniers sont présents, s'élève de 0,1 à 15% en poids, de préférence de 0,3 à 10% en poids, de manière particulièrement préférée de 0,5 à 5% en poids.

4. Procédé selon la revendication 1, caractérisé en ce que la teneur des poudres métalliques en éléments inertes vis-à-vis de l'hydrogénation, lorsque ces derniers sont présents, s'élève de 0 à 10% en poids en ce qui concerne l'aluminium et de 0 à 5% en poids en ce qui concerne chaque élément Si, Ti et C.

5. Procédé selon la revendication 4, caractérisé en ce que la teneur totale en éléments inertes vis-à-vis de l'hydrogénation s'élève de 0 à 15% en poids, de préférence de 0 à 10% en poids.

6. Procédé selon la revendication 1, caractérisé en ce que, quant aux corps moulés, il s'agit de corps moulés possédant une résistance à la pression de 100 à 220 N.

7. Procédé selon la revendication 1, caractérisé en ce que les corps moulés sont de forme cylindrique ou sphériques et possèdent un diamètre de 3 à 7 mm.

8. Procédé selon la revendication 1, caractérisé en ce qu'on effectue l'hydrogénation sous une pression de H₂ de 100 à 400 bar, de préférence de 150 à 300 bar.

9. Procédé selon la revendication 1, caractérisé en ce qu'on travaille avec une quantité de H₂ de 20 à 80 fois molaire.

10. Procédé selon la revendication 1, caractérisé en ce qu'on fait passer l'anhydride maléique à travers le réacteur d'hydrogénation dans un courant ascendant de bas en haut, tandis que l'hydrogène requis pour l'hydrogénation est soit pompé dans le réacteur conjointement avec l'ester insaturé, soit est guidé à contre-courant par rapport à ce dernier, dans un courant de haut en bas.
